# EUROPÄISCHE PATENTSCHRIFT

(11) **EP 1 171 604 B1**
(45) Veröffentlichungstag und Bekanntmachung des Hinweises auf die Patenterteilung: **30.08.2006**
(21) Anmeldenummer: 00926876.4
(22) Anmeldetag: 12.04.2000
(51) Int. Cl.: C12N 15/29, C07K 14/415, A61K 39/36

(54) **DNA-SEQUENZ UND REKOMBINANTE HERSTELLUNG EINES GRAMINAEN-ALLERGENS**
DNA SEQUENCE AND RECOMBINANT PRODUCTION OF A GRAMINAE ALLERGEN
SEQUENCE D'ADN ET PRODUCTION PAR RECOMBINAISON D'UN ALLERGENE DE LA FAMILLE DES GRAMINEES

(30) Priorität: 23.04.1999 DE 19918682
(43) Veröffentlichungstag der Anmeldung: 16.01.2002
(73) Patentinhaber: Merck Patent GmbH, 64293 Darmstadt (DE)
(72) Erfinder: PETERSEN, Arnd, D-23795 Bad Segeberg (DE); SUCK, Roland, D-22303 Hamburg (DE); FIEBIG, Helmut, D-21493 Schwarzenbek (DE); CROMWELL, Oliver, D-21465 Wentorf (DE)
(86) Internationale Anmeldenummer: PCT/EP2000/003259
(87) Internationale Veröffentlichungsnummer: WO 2000/065060

(56) Entgegenhaltungen:
- S VRTALA ET AL: "Immunologic characterization of purified recombinant timothy grass pollen (Phleum pratense) allergens (Phl p 1, Phl p2, Phl p5)" THE JOURNAL OF ALLERGY AND CLINICAL IMMUNOLOGY, Bd. 97, Nr. 3, März 1996 (1996-03), Seiten 781-787, XP000953173 ISSN:0091-6749
- M-F NIOGRET ET AL: "Characterization of pollen polygalacturonase encoded by several cDNA clones in maize" PLANT MOLECULAR BIOLOGY, Bd. 17, Nr. 6, Dezember 1991 (1991-12), Seiten 1155-1164, XP002151351 ISSN:0167-4412
- R SUCK ET AL.: "Complementary DNA cloning and expression of a newly recognized high molecular mass allergen Phl p 13 from timothy grass (Phleum pratense)" CLINICAL AND EXPERIMENTAL ALLERGY, Bd. 30, Nr. 3, März 2000 (2000-03), Seiten 324-332, XP000953168 ISSN:0954-7894
- S FISHER ET AL.: "Characterization of Phl p4, a major timothy grass (Phleum pratense) pollen allergen" THE JOURNAL OF ALLERGY AND CLINICAL IMMUNOLOGY, Bd. 98, Nr. 1, Juli 1998 (1998-07), Seiten 189-198, XP000953216 ISSN:0091-6749

## Beschreibung

Die Erfindung betrifft die Identifizierung und Charakterisierung eines Gräserpollen-Allergens sowie des dafür kodierenden rekombinanten DNA-Moleküls. Als natürlicher Rohstoff dienen die Pollen von Phleum pratense. Die rekombinanten DNA-Moleküle und die abgeleiteten Polypeptide, können zur Therapie von pollenallergischen Krankheiten genutzt werden. Weiterhin können die rekombinant hergestellten Proteine zur Diagnostik von Pollenaltergien genutzt werden.

Allergien vom Typ 1 haben weltweite Bedeutung. Bis zu 20% der Bevölkerung von industrialisierten Länder leiden unter Beschwerden wie allergischer Rhinitis, Konjunktivitis oder Bronchialasthma. Diese Allergien werden durch in der Luft befindliche Allergene (Aeroallergene), die von Quellen unterschiedlicher Herkunft wie Pflanzenpollen, Milben, Katzen oder Hunden freigesetzt werden, hervorgerufen. Bis zu 40% dieser Typ 1-Allergiker wiederum zeigen spezifische IgE-Reaktivität bei Gräserpollen (Freidhoff et al., 1986. J Allergy Clin Immunol 78, 1190-201).

Bei den Typ 1-Allergie auslösenden Substanzen handelt es sich um Proteine, Glykoproteine oder Polypeptide. Diese Allergene reagieren nach Aufnahme über die Schleimhäute mit den bei sensibilisierten Personen an der Oberfläche von Mastzellen gebundenen IgE-Molekülen. Werden zwei IgE-Moleküle durch ein Allergen miteinander vemetzt, führt dies zur Ausschüttung von Mediatoren (z.B. Histamin, Prostaglandinen) und Zytokinen durch die Effektorzelte und damit zu den entsprechenden klinischen Symptomen.

In Abhängigkeit von der relativen Häufigkeit der Allergiker, die IgE-Antikörper gegen bestimmte Allergene aufweisen, wird zwischen Major- und Minorallergenen unterschieden. Im Fall vom Lieschgras (Phleum pratense) sind bislang PhI p 1 (Petersen et al., 1993, J. Allergy Clin. Immunol. 92, 789-796), PhI p 5 (Matthiesen und Löwenstein, 1991, Clin. Exp. Allergy 21, 297-307; Petersen et al., 1992), PhI p 6 (Petersen et al., 1995, Int. Arch. Allergy Immunol. 108, 49-54) und PhI p 2/3 (Dolecek et al., 1993, FEBS 335 (3), 299-304) als Majorallergene und PhI p 4 (Löwenstein, 1978, Prog. Allergy 25, 1-62) sowie Gruppe 10 und 11 aus Lolium perenne (Ansari et. al., 1987, J. Allergy Clin. Immunol. 80, 229-235) als Minorallergene charakterisiert worden.

Im Zusammenhang mit der vorliegenden Erfindung ist das Allergen PhI p 4 von besonderer Bedeutung, da es eine ähnliche Molekularmasse von ca. 55 kDa (Fischer et. al., 1996, J. Allergy Clin Immunol. 98 (1), 189-98) wie das neue Allergen besitzt und damit dem erfindungsgemäß hergestellten Allergen am ehesten vergleichbar ist, sich jedoch immunologisch und biochemisch deutlich unterscheidet. Im Gegensatz zu den anderen o.g. Allergenen ist PhI p 4 das einzige, dessen genomische oder transkriptive (cDNA) Sequenz noch nicht identifiziert ist. Sequenzdaten liegen u.a. vor von PhI p 1 (Laffer et al., 1994, J. Allergy Clin. Immunol. 94, 1190-98; Petersen et al., 1995, J. Allergy Clin. Immunol. 95(5). 987-994), PhI p 5 (Vrtala et al., 1993, J. Immunol. 151 (9), 4773-4781), PhI p 6 (Petersen et al., 1995, Int. Arch. Allergy Immunol. 108 (1), 55-59) und PhI p 2 (Dolecek et. al., 1993, FEBS 335 (3), 299-304). Mit Hilfe von cDNA Sequenzen ist es möglich, rekombinante Allergene herzustellen, die in der Diagnostik und Therapie Verwendung finden können (Scheiner and Kraft, 1995, Allergy 50, 384-391).

Ein klassischer Ansatz zur wirksamen therapeutischen Behandlung von Allergien stellt die Spezifische lmmuntherapie oder Hyposensibilisierung dar (Fiebig, 1995, Allergo J. 4 (6), 336-339, Bousquet et al., 1998, J. Allergy Clin Immunol. 102(4), 558-562). Dabei werden dem Patienten natürliche Allergenextrakte in steigenden Dosen subkutan injiziert. Allerdings besteht bei dieser Methode die Gefahr von allergischen Reaktionen oder sogar eines anaphylaktischen Schocks. Um diese Risiken zu minimieren, werden innovative Präparate in Form von Allergoiden eingesetzt. Dabei handelt es sich um chemisch modifizierte Allergenextrakte, die deutlich reduzierte IgE-Reaktivität, jedoch identische T-Zell-Reaktivität im Vergleich zum nicht behandelten Extrakt aufweisen (Fiebig, 1995, Allergo J. 4 (7), 377-382).

Eine noch weitergehende Therapieoptimierung wäre mit rekombinant hergestellten Allergenen möglich. Definierte, ggfs. auf individuelle Patienten abgestimmte Cocktails von hochreinen rekombinant hergestellten Allergenen könnten Extrakte aus natürlichen Allergenquellen ablösen, da diese außer den verschiedenen Allergenen eine größere Zahl von immunogenen, aber nicht allergenen Begleitproteinen enthalten. Realistische Perspektiven, die zu einer sicheren Hyposenibilisierung mit Expressionsprodukten führen können, bieten gezielt mutierte rekombinante Allergene, bei denen lgE-Epitope spezifisch deletiert werden, ohne die für die Therapie essentiellen T-Zell Epitope zu beeinträchtigen (Schramm et al., 1999, J. Immunol. 162, 2406-2414).

Eine weitere Möglichkeit zur therapeutischen Beeinflussung des gestörten Th-Zell-Gleichgewichtes bei Allergikern ist die Behandlung mit expressionsfähiger DNA, die für die relevanten Allergene kodiert. Erste experimentelle Belege für die allergenspezifische Beeinflussung der Immunantwort konnte an Nagem durch Injektion von Allergen-kodierender DNA erbracht werden (Hsu et al., 1996, Nature Medicine 2 (5), 540-544).

Die Erfindung kann in der In-vitro- und In-vivo-Diagnostik von allergischen Erkankungen, speziell der Pollinosis, vorteilhaft angwendet werden. Dazu wird die klonierte Nukleinsäure in einen Expressionsvektor ligiert und dieses Konstrukt in einem geeigneten Zelltyp expremiert. Nach biochemischer Reinigung steht dieses rekombinante Allergen zur Detektion von IgE-Antikörpern in etablierten Verfahren zur Verfügung. Andererseits kann die Erfindung als eine essentielle Komponente in einem rekombinanten Allergen-haltigen oder Nukleinsäure-haltigen Präparat zur spezifischen Immuntherapie angewendet werden. Hierbei bieten sich mehrere Möglichkeiten. Zum einen kann das in der Primärstruktur unveränderte Protein Bestandteil des Präparates sein. Zum anderen kann die Nukleinsäure an sich, wenn sie mit einem eukaryontischen Expressionsvektor ligiert wird, ein Präparat darstellen, das direkt appliziert den allergischen Immunzustand im therapeutischen Sinne verändert.

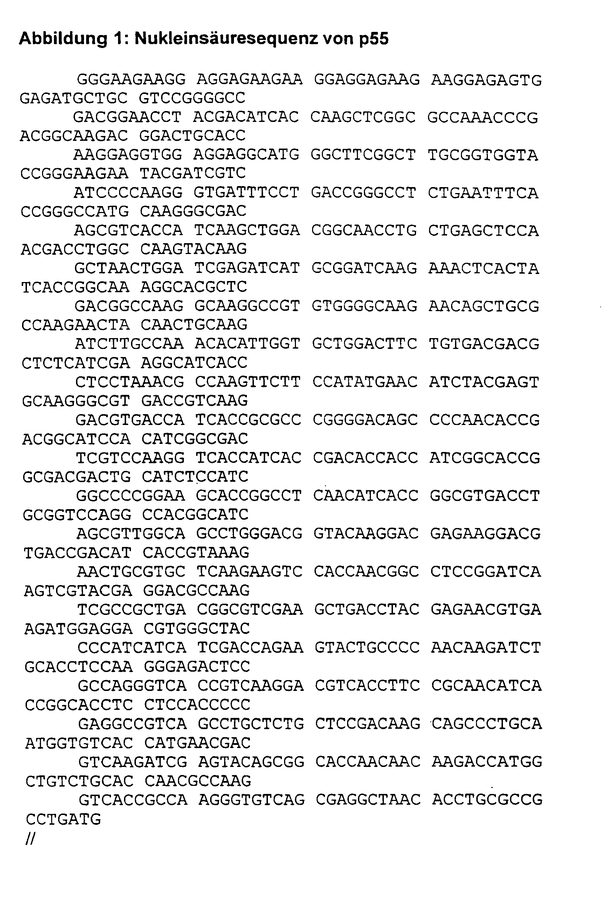

Bei der Erfindung handelt es sich um ein rekombinantes DNA-Molekül, das aus einer Nukleinsätiresequenz (Abb. 1) besteht und für ein Allergen kodiert. Als natürlicher Rohstoff dienen Pollenkömer der Graminaen, wie z.B. Phleum pratense, Lolium perenne, Dactylis glomerata, Poa pratensis, Cynodon dactylon, Holcus tanatus u.a..

Nach der Reinigung und Isolierung des natürlichen Allergens wurde eine N-terminale Proteinsequenzierung vorgenommen. Basierend auf der daraus deduzierten Nuklein-säuresequenz erfolgt die Herstellung eines Primers. Mit Hilfe dieses Primers wurde aus einer cDNA-Population von Pollen mittels PCR die entsprechende cDNA gewonnen, kloniert und charakterisiert.

Nach Expression des rekombinanten DNA-Moleküls mittels geeigneter Expressionsvektoren in zellulären Systemen, wurde das Allergen gereinigt.

Die Reinigung des natürlichen Allergens aus Lieschgraspollen wurde in einem Zweischrittverfahren durchgeführt. Nach der wässrigen Extraktion von Pollen wurde der erhaltene Extrakt mittels Hydrophober-Interaktionschromatographie in zwei Fraktionen getrennt, die Durchlauffraktion und das Eluat. Die Durchlauffraktion enthielt drei Allergene, PhI p 1 (30-35 kDa), PhI p 2/3 (11-14 kDa) und ein unbekanntes Allergen (55-60 kDa). Diese Proteine wurden durch Gelfiltration mit Superdex 75 voneinander getrennt.

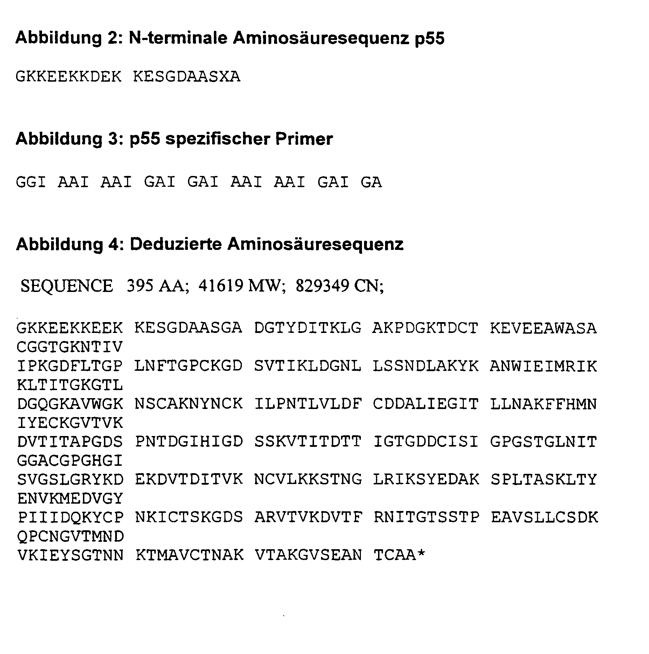

Dieses bisher unbekannte Allergen (Arbeitsbezeichnung p55 ), endgültige Bezeichnung "Phi p 13"; Suck et al., Clin. Exp. Allergy 30 (3), 2000, 324-332 wurde mittels SDS-PAGE aufgetrennt, anschließend auf eine PVDF-Membran geblottet und eine genau definierte Fraktion isoliert. Von diesem p55-Molekül wurde durch Edman-Degradation eine N-terminale Aminosäuresequenz bestimmt (Abb. 2).

Zur Herstellung und Klonierung der korrespondierenden cDNA des p55 wurde erfindungsgemäß ein spezifischer DNA-Primer (21mer) basierend auf der N-terminalen Sequenz Konstruiert (Abb. 3). Als zweiter Primer wurde eine Ankersequenz genutzt, die in dem zur reversen Transkription genutzten Oligo-dT-Primer lokalisiert war. Mit einer cDNA, die aus der repräsentativen mRNA-Population aus Phleum-pratense-Pollen hergestellt wurde, und dem erfindungsgemäßen sowie dem Ankerprimer wurde eine PCR-Reaktion unter stringenten Bedingungen durchgeführt. In der analytischen Gelelektrophorese der PCR-Reaktion wurde ein Amplifikat mit einer Größe von 1.65 kb identifiziert. Dieses Arnplifikat wurde in einen pCR2.1 Vektor ligiert und erfolgreich transformiert. Sequenzierungen der lnserts von zwei verschiedenen Klonen ergaben die identische Sequenz.

In diesem Primäramplifikat wurde ein offenes Leseraster (ORF) von 1492 bp (siehe Abb. 1) identifiziert.

Zur Herstellung des entsprechenden rekombinanten Proteins (Abb. 4) aus dieser Nukleinsäure wurde zunächst eine Umklonierung von dem pCR2.1 Vektor mittels Restriktionsenzymen in den Expressionsvektor pProEx Htb vorgenommen. Nach der Expression und der biochemischen Reinigung des Expressionsproduktes erfolgten mehrere Analysen zur allergenen Beschaffenheit des entwickelten Proteins. In sämtlichen Analysen, z.B. Westem Blot und Dot Blot, reagierte das rekombinante Protein spezifisch mit IgE von Patienten, die die diagnostizierten klinischen Symptome einer Gräserpollenallergie aufweisen. Als Kontrolle wurde das natürliche p55 eingesetzt. Demnach handelt es sich bei dem rekombinanten Protein eindeutig um ein Allergen. Dieses Expressionsprodukt dient damit einer hochspezfischen, verbesserten Diagnostik von Graspollen-Allergikern. Wird die Nukleinsäure, die für p55 kodiert, mit einem humanen Expressionsvektor ligiert, können diese Konstrukte ebenfalls als Präparate zur spezifischen Immuntherapie angewendet werden.

Gegenstand der Erfindung ist somit
(a) ein rekombinantes DNA-Molekül, welches eine Nukleotid-Sequenz des Allergens PhI p 13 aus *Phleum pratense* gemäß SEQ ID NO 1 enthält, die für ein Polypeptid kodiert, das die Wirkung des Allergens PhI p 13 aus *Phleum pratense* aufweist,
(b) ein DNA-Molekül, das eine Nukleotidsequenz besitzt, die komplementär zu der in (a) definierten Nukleotidsequenz des Allergens PhI p 13 aus *Phleum pratense* ist,
(c) ein rekombinanter DNA-Expressionsvektor oder ein Klonierungssystem bestehend aus dem rekombinanten DNA-Molekül, welches eine Nukleotid-Sequenz des Allergens PhI p 13 aus *Phleum pratense* gemäß SEQ ID NO 1 enthält, wie in (a) definiert, funktionell verbunden mit einer Expressionskontrollsequenz,
(d) natürliches PhI p 13 Allergen aus *Phleum pratense,* das von der Nukleinsäure gemäß (a) kodiert wird,
(e) ein Polypeptid, das die Wirkung des Allergens PhI p 13 aus *Phleum pratense* aufweist, das von der Nukleinsäure gemäß (a) kodiert wird,
(f) eine Methode zur Herstellung eines Polypeptides, das die Wirkung des Allergens PhI p 13 aus *Phleum pratense* aufweist, durch Kultivierung von prokaryontischen oder eukaryontischen Zellen, die mit einem Expressionsvektor gemäß (c) transformiert sind, und die Gewinnung des entsprechenden Proteins bzw. Polypeptides aus der Kultur
(g) eine Methode zur Diagnose von Pollenallergien *in vitro* unter Verwendung der Polypeptide gemäß (d) oder (e),
(h) eine pharmazeutische Zubereitung, die ein Polypeptid, das die Wirkung des Allergens PhI p 13 aus *Phleum pratense* aufweist, gemäß (d) oder (e) enthält, zur therapeutischen Behandlung von pollenallergischen Menschen oder Tieren,
(i) die Verwendung eines Polypeptids, das die Wirkung des Allergens PhI p 13 aus *Phleum pratense* aufweist, gemäß (d) oder (e) zur Herstellung eines Medikaments zu Behandlung von pollenallergischen Menschen oder Tieren,
(j) die Verwendung von in (c) definierten Konstrukten, welche eine Nukleotid-Sequenz des Allergens PhI p 13 aus *Phleum pratense* gemäß SEQ ID NO 1 enthalten, zur Herstellung eines Medikaments zur DNA-Vakzinierung pollenallergischer Menschen oder Tiere,
(k) die Verwendung der in (c) definierten Vektoren, welche eine Nukleotid-Sequenz des Allergens PhI p 13 aus *Phleum pratense* gemäß SEQ ID NO 1 enthalten, die immunstimulatorischen DNA-Abschnitte enthält, zur Herstellung eines Medikaments zur DNA-Vakzinierung pollenallergischer Menschen oder Tiere.

Die Erfindung dient somit zur Verbesserung der In-vitro-Diagnostik im Rahmen einer Allergen-Komponenten auflösenden Identifizierung des patientenspeziflschen Sensibilisierungsspektrums. Die Erfindung dient ebenfalls zur Herstellung von deutlich verbesserten Präparaten zur spezifischen Immuntherapie von Gräser-pollenallergien.

## Patentansprüche

1. Ein rekombinantes DNA-Molekül, welches eine Nukleotid-Sequenz des Allergens PhI p 13 aus *Phleum pratense* gemäß SEQ ID NO 1 enthält, die für ein Polypeptid kodiert, das die Wirkung des Allergens PhI p 13 aus *Phleum pratense* aufweist.

2. Ein DNA-Molekül, das eine Nukleotidsequenz besitzt, die komplementär zu der in Anspruch 1 definierten Nukleotidsequenz des Allergens PhI p 13 aus *Phleum pratense* ist.

3. Ein rekombinanter DNA-Expressionsvektor oder ein Klonierungssystem bestehend aus dem rekombinanten DNA-Molekül, welches eine Nukleotid-Sequenz des Allergens PhI p 13 aus *Phleum pratense* gemäß SEQ ID NO 1 enthält, wie in Anspruch 1 definiert, funktionell verbunden mit einer Expressionskontrollsequenz.

4. Natürliches PhI p 13 Allergen aus *Phleum pratense,* das von der Nukleinsäure nach Anspruch 1 kodiert wird.

5. Ein Polypeptid, das die Wirkung des Allergens PhI p 13 aus *Phleum pratense* aufweist, das von der Nukleinsäure nach Anspruch 1 kodiert wird.

6. Eine Methode zur Herstellung eines Polypeptides, das die Wirkung des Allergens PhI p 13 aus *Phleum pratense* aufweist, durch Kultivierung von prokaryontischen oder eukaryontischen Zellen, die mit einem Expressionsvektor gemäß Anspruch 3 transformiert sind, und die Gewinnung des entsprechenden Proteins bzw. Polypeptides aus der Kultur.

7. Eine Methode zur Diagnose von Pollenallergien *in vitro* unter Verwendung der Polypeptide aus den Ansprüchen 4 oder 5.

8. Eine pharmazeutische Zubereitung, die ein Polypeptid, das die Wirkung des Allergens PhI p 13 aus *Phleum pratense* aufweist, nach den Ansprüchen 4 oder 5 enthält, zur therapeutischen Behandlung von pollenallergischen Menschen oder Tieren.

9. Verwendung eines Polypeptids, das die Wirkung des Allergens PhI p 13 aus *Phleum pratense* aufweist, nach den Ansprüchen 4 oder 5 zur Herstellung eines Medikaments zu Behandlung von pollenallergischen Menschen oder Tieren.

10. Verwendung von in Anspruch 3 definierten Konstrukten, welche eine Nukleotid-Sequenz des Allergens PhI p 13 aus *Phleum pratense* gemäß SEQ ID NO 1 enthalten, zur Herstellung eines Medikaments zur DNA-Vakzinierung pollenallergischer Menschen oder Tiere.

11. Verwendung der in Anspruch 3 definierten Vektoren, welche eine Nukleotid-Sequenz des Allergens PhI p 13 aus *Phleum pratense* gemäß SEQ ID NO 1 sowie immunstimulatorische DNA-Abschnitte enthalten, zur Herstellung eines Medikaments zur DNA-Vakzinierung pollenallergischer Menschen oder Tiere.

## Claims

1. A recombinant DNA molecule which contains a nucleotide sequence of the allergen PhI p 13 from *Phleum pratense* as per SEQ ID NO 1 which encodes for a polypeptide which has the action of the allergen PhI p 13 from *Phleum pratense.*

2. A DNA molecule which has a nucleotide sequence which is complementary to the nucleotide sequence of the allergen PhI p 13 from *Phleum prat*ense defined in Claim 1.

3. A recombinant DNA expression vector or a cloning system consisting of the recombinant DNA molecule which contains a nucleotide sequence of the allergen PhI p 13 from *Phleum pratense* as per SEQ ID NO 1, as defined in Claim 1, functionally connected to an expression control sequence.

4. Natural PhI p 13 allergen from *Phleum pratense* which is encoded by the nucleic acid according to Claim 1.

5. A polypeptide which has the action of the allergen PhI p 13 from *Phleum pratense* which is encoded by the nucleic acid according to Claim 1.

6. A method for the preparation of a polypeptide which has the action of the allergen PhI p 13 from *Phleum pratense,* by cultivation of prokaryotic or eukaryotic cells which have been transformed by means of an expression vector according to Claim 3, and isolation of the corresponding protein or polypeptide from the culture.

7. A method for the diagnosis of pollen allergies *in vitro* using the polypeptides from Claims 4 or 5.

8. A pharmaceutical composition which comprises a polypeptide which has the action of the allergen PhI p 13 from *Phleum pratense,* according to Claims 4 or 5, for the therapeutic treatment of pollen-allergic humans or animals.

9. Use of a polypeptide which has the action of the allergen PhI p 13 from *Phleum pratense,* according to Claims 4 or 5, for the preparation of a medicament for the treatment of pollen-allergic humans or animals.

10. Use of constructs defined in Claim 3 which contain a nucleotide sequence of the allergen PhI p 13 from *Phleum pratense* as per SEQ ID NO 1, for the preparation of a medicament for the DNA vaccination of pollen-allergic humans or animals.

11. Use of the vectors defined in Claim 3 which contain a nucleotide sequence of the allergen PhI p 13 from *Phleum pratense* as per SEQ ID NO 1 and immunostimulatory DNA fragments, for the preparation of a medicament for the DNA vaccination of pollen-allergic humans or animals.

## Revendications

1. Molécule d'ADN recombinant qui contient une séquence de nucléotides de l'allergène PhI p 13 à partir de *Phleum pratense* conformément à SEQ ID NO 1 qui code pour un polypeptide ayant l'action de l'allergène PhI p 13 à partir de *Phleum pratense.*

2. Molécule d'ADN qui comporte une séquence de nucléotides qui est complémentaire de la séquence de nucléotides de l'allergène PhI p 13 à partir de *Phleum pratense* tel que défini selon la revendication 1.

3. Vecteur d'expression d'ADN recombinant ou système de clonage constitué par la molécule d'ADN recombinant qui contient une séquence de nucléotides de l'allergène PhI p 13 à partir de *Phleum pratense* conformément à SEQ ID NO 1, tel que défini selon la revendication 1, connecté fonctionnellement à une séquence de commande d'expression.

4. Allergène PhI p 13 naturel à partir de *Phleum pratense* qui est codé par l'acide nucléique selon la revendication 1.

5. Polypeptide qui a l'action de l'allergène PhI p 13 à partir de *Phleum pratense* qui est codé par l'acide nucléique selon la revendication 1

6. Procédé pour la préparation d'un polypeptide qui a l'action de l'allergène PhI p 13 à partir de *Phleum pratense,* par culture de cellules procaryotes ou eucaryotes qui ont été transformées au moyen d'un vecteur d'expression selon la revendication 3, et isolation de la protéine ou du polypeptide correspondant à partir de la culture.

7. Procédé pour le diagnostic d'allergies au pollen *in vitro* en utilisant les polypeptides des revendications 4 ou 5.

8. Composition pharmaceutique qui comprend un polypeptide qui a l'action de l'allergène PhI p 13 à partir de *Phleum pratense,* selon les revendications 4 ou 5, pour le traitement thérapeutique des êtres humains ou des animaux allergiques au pollen.

9. Utilisation d'un polypeptide qui a l'action de l'allergène PhI p 13 à partir de *Phleum pratense,* selon les revendications 4 ou 5, pour la préparation d'un médicament pour le traitement des êtres humains ou animaux allergiques au pollen.

10. Utilisation de constructions définies selon la revendication 3 qui contiennent une séquence de nucléotides de l'allergène PhI p 13 à partir de *Phleum pratense* conformément à SEQ ID NO 1, pour la préparation d'un médicament pour la vaccination ADN d'êtres humains ou d'animaux allergiques au pollen.

11. Utilisation des vecteurs selon la revendication 3 qui contiennent une séquence de nucléotides de l'allergène PhI p 13 à partir de *Phleum pratense* conformément à SEQ ID NO 1 et des fragments d'ADN immuno-stimulateurs, pour la préparation d'un médicament pour la vaccination ADN d'êtres humains ou d'animaux allergiques au pollen.


### SEQUENZPROTOKOLL

<110> Merck Patent GmbH
<120> DNA-Sequenz und rekombinante Herstellung eines Graminaen-Allergens
<130> DNA-Sequenz
<140>
   <141>
<160> 4
<170> PatentIn Ver. 2.1
<210> 1
   <211> 1187
   <212> DNA
   <213> Gramineae
<400> 1
   gggaagaagg aggagaagaa ggaggagaag aaggagagtg gagatgctgc gtccggggcc 60
   gacggaacct acgacatcac caagctcggc gccaaacccg acggcaagac ggactgcacc 120
   aaggaggtgg aggaggcatg ggcttcggct tgcggtggta ccgggaagaa tacgatcgtc 180
   atccccaagg gtgatttcct gaccgggcct ctgaatttca ccgggccatg caagggcgac 240
   agcgtcacca tcaagctgga cggcaacctg ctgagctcca acgacctggc caagtacaag 300
   gctaactgga tcgagatcat gcggatcaag aaactcacta tcaccggcaa aggcacgctc 360
   gacggccaag gcaaggccgt gtggggcaag aacagctgcg ccaagaacta caactgcaag 420
   atcttgccaa acacattggt gctggacttc tgtgacgacg ctctcatcga aggcatcacc 480
   ctcctaaacg ccaagttctt ccatatgaac atctacgagt gcaagggcgt gaccgtcaag 540
   gacgtgacca tcaccgcgcc cggggacagc cccaacaccg acggcatcca catcggcgac 600
   tcgtccaagg tcaccatcac cgacaccacc atcggcaccg gcgacgactg catctccatc 660
   ggccccggaa gcaccggcct caacatcacc ggcgtgacct gcggtccagg ccacggcatc 720
   agcgttggca gcctgggacg gtacaaggac gagaaggacg tgaccgacat caccgtaaag 780
   aactgcgtgc tcaagaagtc caccaacggc ctccggatca agtcgtacga ggacgccaag 840
   tcgccgctga cggcgtcgaa gctgacctac gagaacgtga agatggagga cgtgggctac 900
   cccatcatca tcgaccagaa gtactgcccc aacaagatct gcacctccaa gggagactcc 960
   gccagggtca ccgtcaagga cgtcaccttc cgcaacatca ccggcacctc ctccaccccc 1020
   gaggccgtca gcctgctctg ctccgacaag cagccctgca atggtgtcac catgaacgac 1080
   gtcaagatcg agtacagcgg caccaacaac aagaccatgg ctgtctgcac caacgccaag 1140
   gtcaccgcca agggtgtcag cgaggctaac acctgcgccg cctgatg                1187
<210> 2
   <211> 20
   <212> PRT
   <213> Künstliche Sequenz
<220>
   <223> Beschreibung der künstlichen Sequenz: p55 spezifischer primer
<400> 2
   Gly Lys Lys Glu Glu Lys Lys Asp Glu Lys Lys Glu Ser Gly Asp Ala
    1               5                   10                  15
   Ala Ser Xaa Ala
               20
<210> 3
   <211> 26
   <212> DNA
   <213> Künstliche Sequenz
   <220>
   <223> Beschreibung der künstlichen Sequenz:p55 spezifischer primer
<220>
   <221> variation
   <222> (3)
   <223> n = Inosine
<220>
   <221> variation
   <222> (6)
   <223> n = Inosine
<220>
   <221> variation
   <222> (9)
   <223> n = Inosine
<220>
   <221> variation
   <222> (12)
   <223> n = Inosin
<220>
   <221> variation
   <222> (15)
   <223> n = Inosin
<220>
   <221> variation
   <222> (18)
   <223> n = Inosine
<220>
   <221> variation
   <222> (21)
   <223> n = Inosine
<220>
   <221> variation
   <222> (24)
   <223> n = Inosine
<400> 3
   ggnaanaang anganaanaa nganga 26
<210> 4
   <211> 394
   <212> PRT
   <213> Gramineae
<400> 4
   Gly Lys Lys Glu Glu Lys Lys Glu Glu Lys Lys Glu Ser Gly Asp Ala
    1               5                  10                  15
   Ala Ser Gly Ala Asp Gly Thr Tyr Asp Ile Thr Lys Leu Gly Ala Lys
               20                  25                  30
   Pro Asp Gly Lys Thr Asp Cys Thr Lys Glu Val Glu Glu Ala Trp Ala
           35                  40                  45
   Ser Ala Cys Gly Gly Thr Gly Lys Asn Thr Ile Val Ile Pro Lys Gly
       50                  55                  60
   Asp Phe Leu Thr Gly Pro Leu Asn Phe Thr Gly Pro Cys Lys Gly Asp
   65                  70                  75                  80
   Ser Val Thr Ile Lys Leu Asp Gly Asn Leu Leu Ser Ser Asn Asp Leu
                   85                  90                  95
   Ala Lys Tyr Lys Ala Asn Trp Ile Glu Ile Met Arg Ile Lys Lys Leu
               100                 105                 110
   Thr Ile Thr Gly Lys Gly Thr Leu Asp Gly Gln Gly Lys Ala Val Trp
           115                 120                 125
   Gly Lys Asn Ser Cys Ala Lys Asn Tyr Asn Cys Lys Ile Leu Pro Asn
       130                 135                 140
   Thr Leu Val Leu Asp Phe Cys Asp Asp Ala Leu Ile Glu Gly Ile Thr
   145                 150                 155                 160
   Leu Leu Asn Ala Lys Phe Phe His Met Asn Ile Tyr Glu Cys Lys Gly
                   165                 170                 175
   Val Thr Val Lys Asp Val Thr Ile Thr Ala Pro Gly Asp Ser Pro Asn
               180                 185                 190
   Thr Asp Gly Ile His Ile Gly Asp Ser Ser Lys Val Thr Ile Thr Asp
           195                 200                 205
   Thr Thr Ile Gly Thr Gly Asp Asp Cys Ile Ser Ile Gly Pro Gly Ser
       210                 215                 220
   Thr Gly Leu Asn Ile Thr Gly Gly Ala Cys Gly Pro Gly His Gly Ile
   225                 230                 235                 240
   Ser Val Gly Ser Leu Gly Arg Tyr Lys Asp Glu Lys Asp Val Thr Asp
                   245                 250                 255
   Ile Thr Val Lys Asn Cys Val Leu Lys Lys Ser Thr Asn Gly Leu Arg
               260                 265                 270
   Ile Lys Ser Tyr Glu Asp Ala Lys Ser Pro Leu Thr Ala Ser Lys Leu
           275                 280                 285
   Thr Tyr Glu Asn Val Lys Met Glu Asp Val Gly Tyr Pro Ile Ile Ile
       290                 295                 300
   Asp Gln Lys Tyr Cys Pro Asn Lys Ile Cys Thr Ser Lys Gly Asp Ser
   305                 310                 315                 320
   Ala Arg Val Thr Val Lys Asp Val Thr Phe Arg Asn Ile Thr Gly Thr
                   325                 330                 335
   Ser Ser Thr Pro Glu Ala Val Ser Leu Leu Cys Ser Asp Lys Gln Pro
               340                 345                 350
   Cys Asn Gly Val Thr Met Asn Asp Val Lys Ile Glu Tyr Ser Gly Thr
           355                 360                 365
   Asn Asn Lys Thr Met Ala Val Cys Thr Asn Ala Lys Val Thr Ala Lys
       370                 375                 380
   Gly Val Ser Glu Ala Asn Thr Cys Ala Ala
   385                 390